# EUROPÄISCHE PATENTANMELDUNG

(11) **EP 1 564 298 A1**
(43) Veröffentlichungstag der Anmeldung: **17.08.2005**
(21) Anmeldenummer: 04003293.0
(22) Anmeldetag: 13.02.2004
(51) Int. Cl.: C12P 7/42, C12N 9/88, C12N 15/70

(54) **Enzymatische asymmetrische Decarboxylierung von disubstituierten Malonsäuren**

(71) Anmelder: BioSpring GmbH, 60386 Frankfurt (DE)
(72) Erfinder: Aygün, Hüseyin, Dr., 60322 Frankfurt am Main (DE); Wojczewski, Sylvia Dr., 65812 Bad Soden/Taunus (DE); Kircher, Markus, Dr., 60318 Frankfurt am Main (DE); Rosmus, Susann, Dr., 60388 Frankfurt am Main (DE)
(74) Vertreter: Keller, Günter, Dr.

(57) **Zusammenfassung**

Offenbart wird ein Verfahren zur stereoselektiven Decarboxylierung von Malonsäurederivaten der Formel (I) 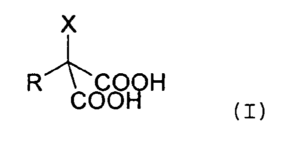 worin X die Bedeutung OH, SH, C2-C10 Alkyl, C1-C10 Alkyloxy, NH₂ hat und R die Bedeutung Aryl-, Heteroaryl-, kondensierte Aryl- sowie kondensierte Heteroarylrest darstellt, der gegebenenfalls substituiert sein kann mit Halogenen, Alkyl-, Alkenyl-, Alkinylresten, Aryl, Furyl, Thiofuryl oder Naphthyl bedeuten kann, zu einer Verbindung der Formel (II) 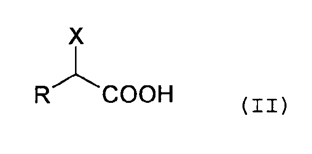 wobei X und R die oben angegebene Bedeutung haben, mit mutierten Decarboxylasen.

## Beschreibung

In der chemisch-pharmazeutischen Industrie zeigt sich immer mehr das Problem, dass bestimmte Substanzen als reine Substanzen, d.h. als nicht racemische Gemische zum Einsatz kommen müssen. Zum Teil stehen jedoch für diese Art der Synthesen keine einfachen Synthesestrategien zu Verfügung. Daher wurden in den letzten Jahren eine ganze Reihe von unterschiedlichsten Enzymen verwendet, die oftmals in ihrer Umsetzung ein bestimmtes Substrat stereo- und regioselektiv bevorzugen, so dass manche Umsetzungen durch den Einsatz von Enzymen wirtschaftlich erst sinnvoll werden.

Bei α-substituierten Malonsäurederivaten, insbesondere aromatischen oder durch Konjugation damit verbundenen Derivaten, steht noch immer nur ein sehr begrenztes Enzymspektrum zur Verfügung. So ist beispielsweise bis heute kein einfaches, heterolog zu exprimierendes Enzym beschrieben, das aromatische oder durch Konjugation damit verbundene α-Hydroxymalonsäuren/Tartronsäure und deren Derivate akzeptiert bzw. das für eine einfache Umsetzung eingesetzt werden kann. Die Decarboxylierung kann schematisch dargestellt werden wie folgt:

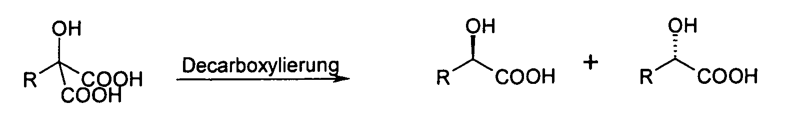

Bisher wurden für die Synthese von aromatischen α-Hydroxycarbonsäuren eine Reihe von enzymatischen Wegen beschrieben, die sich jedoch grundsätzlich von dem hier offenbarten Syntheseweg unterscheiden. Es sind verschiedene Möglichkeiten bekannt, wie auf einem enzymatischen Weg enatiomerenreine aromatische α-Hydroxycarbonsäuren hergestellt werden können [Gröger, A. (2001): Adv.Synth.Catal. 343, 547-558].

Alle bekannten Ansätze zur enzymatischen Synthese von α-Hydroxycarbonsäuren haben den Nachteil, dass entweder HCN eingesetzt werden muss, oder häufig ein theoretisches Ausbeutemaximum von nur 50 % erreichbar ist oder zusätzlich Reduktionsäquivalente regeneriert werden müssen.

Allen Verfahren gemeinsam ist, dass nie eine Dicarbonsäure als Ausgangssubstanz eingesetzt wird, die dann gezielt, asymmetrisch decarboxyliert wird, so dass nur das eine Enantiomer der gesuchten Verbindung entsteht.

Die einzelnen vorbekannten Synthesestrategien sollen im folgenden kurz vorgestellt werden:

Verschiedene Wege gehen von racemischen Gemischen von α-OH-Estern oder O-acetylierten Cyanhydrinen aus, die dann selektiv hydrolysiert werden. Für die O-Acyl geschützten Mandelsäurester wird z.B. die Penicillin Amidase aus *Alcaligenes faecalis* eingesetzt, wenn das (S)-Enantiomer erzeugt werden soll [C. Fuganti, C.M. Rosell, S. Servi, A. Tagliani, M. Terreni, Tetrahedron: Asymmetry 1992, 3, 383]. (R)-Enantiomere sperriger Carbonsäureester sind hingegen mittels einer Protease aus *Aspergillus oryzae* leicht zugänglich. Die entsprechenden Produkte müssen häufig über Kristallisation weiter aufgereinigt werden um eine höhere Produktreinheit zu erhalten.

Bei der Verwendung von O-Acyl-Cyanhydrinen nutzt man den Umstand aus, dass die nicht deacylierten Cyanhydrine leicht in die entsprechenden Carbonsäuren überführt werden können, ohne die optische Drehung der Verbindung zu verändern. Man kann also von racemischen Gemischen von O-Acyl-Cyanhydrinen ausgehen, die dann selektiv mit entsprechenden Lipasen (z.B. aus *Pseudomonas spec.* oder *Arthrobacter spec.)* hydrolysiert werden.

Ein weiterer Syntheseweg, ausgehend von racemischen Cyanhydrinen, besteht in der Möglichkeit, die Cyanhydrine direkt enantioselektiv mittels Nitrilasen, beispielsweise aus *Rhodococcus* zu hydrolysieren, was zu einer einfacheren Synthesestrategie führt, da in einem Schritt die enantiomerenreine Carbonsäure entsteht. Da auch diese Strategie auf einer Racematspaltung beruht, ist das Ausbeutemaximum ebenfalls auf 50 % begrenzt.

Einen ähnlichen, aber umgekehrten Weg setzt man zur enantioselektiven Synthese von Cyanhydrinen ein. Ausgehend vom Aldehyd (z.B. Benzaldehyd) wird mittels Oxynitrilasen eine enzymkatalysierte Blausäureaddition durchgeführt, welche die entsprechenden Cyanhydrine liefert (z.B. Mandelonitril). Dabei sind oftmals die Reaktionsbedingungen entscheidend, die eine enantiomerenreine Umsetzung ermöglichen.

Für die bis hierher aufgeführten Synthesestrategien ist jedoch immer erst eine Modifikation der Ausgangssubstanz (Veresterung der OH-Funktion oder der Carbonsäurefunktion) nötig, um im Anschluss die Enantiomeren trennen zu können. Die anderen Synthesestrategien verwenden oder erzeugen Blausäure, was wiederum eine deutliche Einschränkung in der Durchführbarkeit darstellt.

Ein weiterer Zugang zu α-Hydroxycarbonsäuren besteht in der enantioselektiven Reduktion prochiraler α-Ketocarbonsäuren bzw. der entsprechenden Ester mittels Dehydrogenasen. Wenn nicht Gesamtzellen zum Einsatz kommen, muss der Einsatz von Dehydrogenasen an ein weiteres, Reduktionsäquivalente regenerierendes System gekoppelt werden, was die Einsatzmöglichkeiten wiederum stark einschränkt. Um den Verbrauch von Reduktionsäquivalenten nicht als Limitation auftreten zu lassen, kommen oftmals auch gekoppelte Enzym/Redox-Prozesse zum Einsatz, in denen die verbrauchten Reduktionsequivalente enzymatisch regeneriert werden.

Die hier offenbarte stereoselektive Synthese beschreibt einen neuen Weg. Ausgehend von einem Malonsäurederivat wird eine Carboxylgruppe enzymatisch abgespalten, so dass nur ein Enantiomer erzeugt wird. Diese Synthesestrategie bringt den großen Vorteil mit sich, dass weder die Ausgangssubstanzen zunächst verestert werden müssen, noch, dass die gewünschten Carbonsäuren zunächst in Cyanhydrine (HCN-Einsatz) überführt werden müssen. Außerdem ist es zumindest theoretisch möglich eine Produktausbeute von 100 % zu erreichen.

Ohta [Advances in Biochemical Engineering/Biotechnology, Vol. 63, S. 1-29, Springer Verlag (1999)] beschreibt ein Enzym [Arylmalonat-Decarboxylase (AMDase)] aus *Alcaligenes bronchisepticus* KU1201, welches α-Methyl-Phenylmalonsäuren asymmetrisch zu den entsprechenden Phenylessigsäurederivaten decarboxyliert.

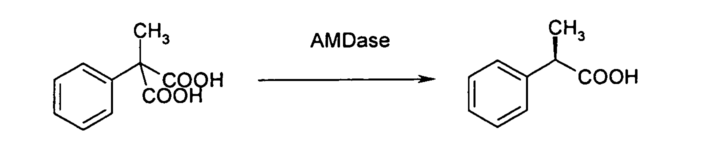

Mit Hilfe von Mutanten stellte Ohta fest, daß die Cysteinreste, insbesondere der Rest an Position 188, an der Katalyse beteiligt sind. Bei diesen Versuchen wurden die vier Cysteinreste an den Positionen 101, 148, 171 bzw. 188 ersetzt durch Serinreste.

Im Rahmen der vorliegenden Anmeldung wird die gezielte gentechnische Veränderung des Enzymes Arylmalonat-Decarboxylase (AMDase) offenbart. Durch die offenbarten Mutationen ist es möglich, auch aromatische Tartronsäurederivate in hoher Ausbeute und mit hohem enantiomeren Überschuß durch asymmetrische Decarboxylierung zu produzieren. Es ist also möglich, mit den mutierten Enzymen Substrate zu decarboxylieren, die mit dem Wildtyp-Enzym nicht decarboxyliert werden können.

Die offenbarten mutierten Decarboxylasen können Substrate mit einer deutlich höheren Enzymaktivität umsetzen. Dies ist für die technische Verwendung von großer Bedeutung.

In einem weiteren Aspekt der vorliegenden Erfindung werden solche mutierten Decarboxylasen offenbart, die darüber hinaus eine signifikante Verbesserung der thermischen Stabilität aufweisen, was insbesondere die technische Anwendbarkeit der erfindungsgemäßen Enzyme als Biokatalysatoren wesentlich erhöht.

Gegenstand der vorliegenden Erfindung ist daher ein Verfahren zur stereoselektiven Decarboxylierung von Malonsäurederivaten der Formel (I) 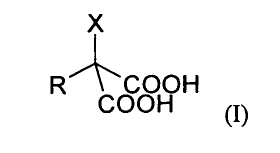 worin X die Bedeutung OH, SH, C2-C10 Alkyl, C1-C10 Alkyloxy, NH₂ hat und R die Bedeutung Aryl-, Heteroaryl-, kondensierte Aryl- sowie kondensierter Heteroarylrest darstellt, der gegebenenfalls substituiert sein kann mit Alkyl-, Alkenyl-, Alkinylresten oder Halogenen. Unter Arylresten im Sinn der vorliegenden Anmeldung werden aromatische Kohlenwasserstoffreste, wie beispielsweise der Phenyl-, Naphthyl- oder Anthrylrest verstanden. Heteroarylreste sind aromatische Kohlenwasserstoffreste, die neben Kohlenstoffatomen auch Heteroatome, und zwar insbesondere Stickstoff, Sauerstoff oder Schwefel enthalten können. Beispielhaft können hier genannt werden die Furyl-, Pyrrolyl-, Thiophenyl-, Pyrazolyl-, Imidazolyl-, Pyridinyl-, Pyridazinyl-, Pyrimidinyl-, Pyrazinyl-, Indolyl-, Purinyl-, Chinolinyl-, Benzofuranyl, Carbazolyl-, Benzothiophenyl-, Isochinolinyl-, Chinoxalinyl-, Pteridinyl-, Thiazolyl-, Oxozolyl- oder Acridinyl-Reste. Diese aromatischen Reste können gegebenenfalls auch an verschiedenen Stellungen substituiert sein durch Halogene, Alkyl, Alkenyl, Alkinyl oder Alkoxyreste. Bei der Umsetzung mit einer Decarboxylase entsteht eine Verbindung der Formel (II) 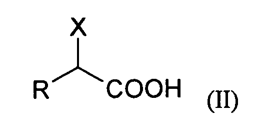 wobei X und R die oben angegebene Bedeutung haben.

In einer bevorzugten Ausführungsform eröffnen die offenbarten Decarboxylasen einen wichtigen Zugang zu der bedeutenden Klasse der chiralen substituierten Essigsäuren. Hierzu gehören beispielsweise insbesondere jene Verbindungen, die neben unterschiedlichen Substituenten am aromatischen Ringsystem, substituierte Phenyl-, Naphthyl-, Furyl- und Thiofuryl-Ringsysteme enthalten können. Beispielhafte Strukturen sind nachfolgend dargestellt: 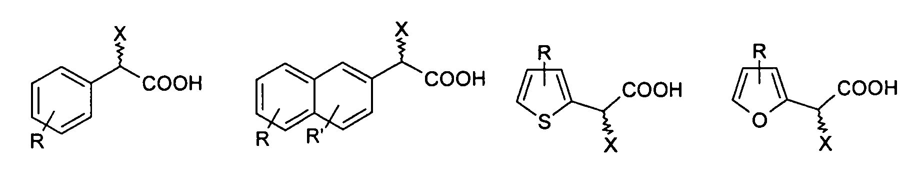 R und R' stehen hier für Substituent der Arylreste.

Die Malonsäure-Derivate (Formel I) lassen sich entweder direkt aus der Malonsäure aufbauen oder sind durch Carboxylierung der entsprechenden Monocarbonsäuren erhältlich.

In einem weiteren Aspekt betrifft die vorliegende Erfindung Decarboxylasen enthaltend eine Aminosäuresequenz entsprechend der SEQ ID NO:1, die wenigstens eine Mutation gegenüber der SEQ ID NO:1 aufweist, wobei die Mutuation an einer der folgenden Aminosäurepositionen vorliegt: 17, 19, 22, 25, 32, 41, 42, 45, 47, 53, 60, 61, 63, 68, 74, 84, 85, 87, 94, 103, 112, 119, 121, 139, 142, 155, 168, 173, und/oder 178 , wobei die an der Aminosäureposition in SEQ ID NO:1 angegebene Aminosäure ersetzt ist durch eine beliebige andere Aminosäure, mit der Maßgabe, daß an der Position 188 der Cysteinrest nicht ersetzt sein darf durch andere Aminosäuren sowie Insertions- und/oder Deletionsmutanten, wobei nicht mehr als jeweils 10 Aminosäuren in die Sequenz gemäß SEQ ID NO:1 eingefügt und/oder deletiert sein dürfen.
Durch Mutation bestimmter, erfindungsgemäß aufgefundener Aminosäurereste können Decarboxylasevarianten bereitgestellt werden, die nun auch in der Lage sind, neue Substratgruppen, wie Beispielsweise α-C₂₋₁₀-Alkyl-, α-Hydroxy- , α-Thiol- und α-Amino-Phenylmalonsäuren sowie deren Derivate asymmetrisch zu decarboxylieren. Diese Neuerung eröffnet einen wertvollen Zugang zu chiralen α-Alkyl-, α-Hydroxy-, α-Thiol- und α-Amino-Phenylessigsäuren als auch zu deren Derivaten. Bevorzugte bespielhafte Produktstrukturen sind nachfolgend aufgeführt: 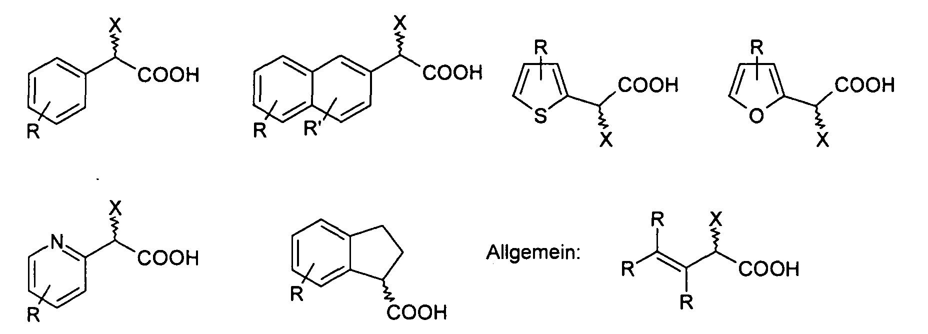 X: Alkyl, OH, NH₂ und SH; R: Halogen, Alkyle-, Alkenyle-, Alkinyle-, Aryle-, Thioether- und Ether.

Bevorzugt sind Decarboxylasen, die eine Aminosäuresequenz mit wenigstens einer Mutation gegenüber der SEQ ID NO:1 aufweisen, bei der die Mutation an folgenden Aminosäurepositionen vorliegt: 17, 19, 32, 41, 42, 45, 47, 53, 60, 61, 63, 68, 74, 84, 85, 87, 94, 103, 119, und/oder 142, und besonders bevorzugt sind solche Decarboxylasen mit einer Mutation an einer der folgenden Positionen: 19, 32, 42, 45, 47,60, 61, 63, 68, 84, 85, und/oder 103, wobei die in SEQ ID NO:1 an dieser Position angegebene Aminosäure ersetzt ist durch Glycin oder Alanin.

In einem weiteren Aspekt der vorliegenden Erfindung wurde überraschenderweise festgestellt, daß die Thermostabilität der erfindungsgemäßen Decarboxylase durch spezielle Mutationen deutlich verbessert werden kann. Eine Verbesserung der Thermostabilität wird vor allem dann erhalten, wenn die in SEQ ID NO:1 angegebene Aminosäure an den Positionen 15, 32, 74, 75, 128, 163, 165 und/oder 171 ersetzt wird durch Alanin oder Glycin.

Von Miyamoto und Ohta wurden die enzymspezifischen Parameter wie pH-Optimum und Temperaturoptimum bestimmt und publiziert [Miyamoto, K. & Ohta, H. (1992: Eur.J.Biochem. 210, 475-481)]. Zur Ermittlung der Substratspezifität der isolierten AMDase wurden von Ohta et al. eine Reihe von Untersuchungen durchgeführt, in denen die akzeptierten Substrate und die Spezifität des Enzyms klar aufgezeigt wurde.

Auch konnten Ohta et al. zeigen, dass die entstandenen Verbindungen (R)-Konfiguration aufweisen. Wird jedoch Glycin 74 zu einem Cystein mutiert und gleichzeitig das aktive Cystein 188 zu einem Serin mutiert, wird die Produktkonfiguration verändert, es entstehen dann (S)-konfigurierte Verbindungen (Terao,Y. et al. Chem. Listy **97**, 488 (2003)). Diesen Effekt kann man auch erreichen, wenn bestimmte Aminosäuren, die benachbart zu Glycin 74 liegen zu einem Cystein mutiert werden. Werden beide Positionen (74 und 188) mit Cysteinseitenketten eingesetzt, entsteht eine Decarboxylase, die die Ausgangsverbindungen in Monocarbonsäuren mit unterschiedlicher Geschwindigkeit umsetzt. Von Ohta et al. wurden auch noch eine Reihe weiterer Mutationen der anderen Cysteine im Enzym beschrieben, die zu einer Veränderung der Umsatzgeschwindigkeiten der AMDase, bezogen auf die Phenylmalonsäure und deren Derivate, führen. Jedoch wurden bisher keine weiteren Substrate als α-Methyl- bzw. nicht weiter am α-Kohlenstoffatom substituierte Arylmalonsäuren (schon die Einbringung einer α-Ethylfunktion führt zu einem Einbruch der Enzymaktivität) umgesetzt.

In einem weiteren Aspekt betrifft die vorliegende Erfindung daher auch Decarboxylasen, bei denen ein Cysteinrest an der Position 188 der SEQ ID NO:1 nicht mutiert ist und die bei der stereoselektiven Decarboxylierung zu Verbindungen der Formel (II) führt, die die R-Konfiguration aufweisen.

In einer weiteren Ausführungsform betrifft die vorliegende Erfindung Decarboxylasen, bei deren Einsatz Verbindungen der Formel (II) mit der S-Konfiguation entstehen. Bei diesen Decarboxylasen wird eine Aminosäure zwischen den Positionen 69 und 81 der SEQ ID NO:1 ersetzt ist durch ein Cystein und gleichzeitig der Cysteinrest an Position 188 ersetzt ist durch eine andere Aminosäure als Cystein.

In einer weiteren bevorzugten Ausführungsform werden erfindungsgemäße Decarboxylasen mit einer deutlichen Verbesserung der Umsetzung von Malonsäure-Derivaten der Formel III 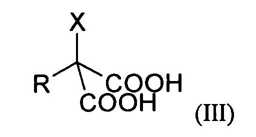 zu Verbindungen der Formel (IV) 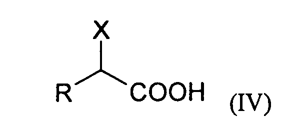 offenbart, worin X die Bedeutung F, CH₃ oder H hat und R die Bedeutung substituierte und nicht-substituierte Aryl-, Heteroaryl-, kondensierte Aryl- sowie kondensierte Heteroarylrest hat, wobei der Substituent ein Halogen, Alkyl-, Alkenyl-, Alkinylrest sein kann.

Bei diesen Decarboxylasen ist wenigstens eine der in SEQ ID NO:1 angegebenen Aminosäuren an den Positionen 17, 19, 22, 25, 32, 41, 42, 45, 47, 53, 60, 61, 63, 68, 74, 84, 85, 87, 94, 103, 112, 119, 121, 139, 142, 155, 168, 173, und/oder 178 ersetzt.

Die erfindungsgemäßen Decarboxylasen können am C-Terminus und/oder am N-Terminus weitere Aminosäuren aufweisen. Bei diesen zusätzlichen Aminosäuresequenzen kann es sich um solche handeln, die durch die Klonierung entstanden sind oder die Vorteile bei der Herstellung, Reinigung oder beim Einsatz bringen. Die Anfügung einer Polyhistidinsequenz (Hexa-his) am C- oder N-Terminus ermöglicht die Reinigung des Enzyms. Es ist aber auch möglich, eine Leader-Sequenz vor das Gen kodierend für die mutierte Decarboxylase in den Vektor einzubauen. Dadurch kann nach der Expression das Enzym leicht aus der Wirtszelle ausgeschleust werden und es ist eine verbesserte Herstellbarkeit des Enzyms dadurch möglich. Alternativ hierzu ist es auch möglich, am C- oder N-Terminus der Decarboxylase solche Strukturen anzuschließen, die dazu führen, daß das Enzym nach Expression mit der Zellwand des Wirtsorganismus verbunden bleibt, so daß die Verwendung von Wirtsorganismen, die erfindungsgemäße Decarboxylasen an den Zelloberflächen tragen, als Biokatalysatoren ermöglicht wird.

Die erfindungsgemäßen Decarboxylasen können in bevorzugter Weise mit Hilfe von rekombinanten Techniken hergestellt werden. Hierzu wird üblicherweise ein Vektor, der die erforderlichen Bestandteile zur Expression einer fremden Nucleotidsequenz enthält, in einen geeigneten Wirtsorganismus eingebracht. Der Vektor beinhaltet an geeigneter Stelle die Polynucleotidsequenz mit der SEQ ID NO:2 und die für die Mutation bzw. die Mutationen kodierenden Basenaustausche. Weiterhin ist es möglich, daß der Wirtsorganismus das Gen gegebenenfalls mit geeigneten Regulationselementen wie beispielsweise Promotor etc. in dem Chromosom integriert enthält. Möglich ist auch, daß der Wirtsorganismus das Fremdgen sowohl im Vektor, wie auch im Chromosom integriert enthält.

Die Decarboxylase kann je nach Einsatzzweck, Wirtsorganismus und Vektor im cytoplasmatischen Raum des Wirtsorganismus exprimiert, in den periplasmatischen Raum transportiert oder aus der Zelle in das umgebende Medium ausgeschleust werden. Die erfindungsgemäßen Decarboxylasen können anschließend , soweit dies erforderlich ist, durch übliche Reinigungsverfahren gereinigt werden, wobei der Grad der Reinheit von dem beabsichtigten Verwendungszweck abhängt.
Für die Expression der erfindungsgemäßen Decarboxylasen können übliche Wirtsorganismen verwendet werden wie beispielsweise *Escherichia coli, Bacillus subtilis, Saccharomyces cerevisiae, Hansenula spec., Pichia pastoria* oder andere prokaryontische oder eukaryontische Expressionssysteme. Bevorzugt werden die erfindungsgemäßen Decarboxylasen in *Escherichia coli* exprimiert.

Ein weiterer Aspekt der vorliegenden Erfindung betrifft transformierte Wirtsorganismen, die die erfindungsgemäßen Decarboxylasen exprimieren können. Je nach beabsichtigtem Verwendungszweck ist es möglich, für die Decarboxylierung die ganzen transformierten Wirtszellen einzusetzen. Es ist aber auch möglich, Aufschlüsse bzw. Lysate oder Lagerformen (z.B. getrocknete Zellen) dieser Zellen, die die Decarboxylase enthalten, einzusetzen. Diese Aufschlüsse können teilweise gereinigt sein. Weiterhin ist es möglich, die Decarboxylasen an feste Substrate zu koppeln, um Biokatalysatoren für die kontinuierliche Umsetzung bereitzustellen. Bevorzugt können die Decarboxylasen an Säulenmaterial gekoppelt oder in Polymere eingeschlossen werden. Die Substrate können dann in flüssiger Form durch die erfindungsgemäße Decarboxylasen enthaltenden Säulen geführt werden und die stereoselektiv decarboxylierten Produkte können nach dem Durchlauf durch die Säule gereinigt werden.

Die vorliegende Erfindung offenbart also
- a): die gentechnische Veränderung des Enzyms AMDase aus *Alcaligenes bronchisepticus* KU1201 derart, dass nun, neben einer deutlich verbesserten Umsetzung der bisher bekannten Substrate, auch aromatische Tartronsäurederivate in hoher Ausbeute und mit hohem Enantiomerenüberschuß durch asymmetrische Decarboxylierung produziert werden können,
- b): eine optimierte DNA-Sequenz, die zu deutlich verbesserter Expression des Enzymes führt,
- c): zahlreiche zuvor nicht beschriebene Enzymmutanten, die die Substrate mit einer deutlich höheren Enzymaktivität umsetzen können, und
- d): Mutationen, die die thermische Stabilität des Enzyms signifikant verbessern und so, mit den obigen Vorteilen, die technische Nutzbarkeit des Enzyms als Biokatalysator erhöhen.

Die erfindungsgemäßen Arylmalonatdecarboxylasen (AMDase, E.C. 4.1.1.76), die gegenüber SEQ ID NO:1 mutiert sind, erfüllen die Anforderungen an ein technisch einsetzbares Enzym. Es lässt sich leicht in einfachen Mikroorganismen heterolog exprimieren und im Gegensatz zu vielen bisher beschriebenen Decarboxylasen benötigt dieses Enzym kein Coenzym, um eine selektive Decarboxylierung zu bewerkstelligen, weil eine Cysteinseitenkette als aktiver Rest verwendet wird. Diese AMDase akzeptiert nicht nur aromatische Malonsäurederivate. Toleriert werden auch bestimmte Substituenten im aromatischen Ringsystem, sowie α-Methyl als auch einige α-Halogenide als Substrat. Derivate, die am α-Kohlenstoffatom eine Hydroxyl-, Thiol-, Alkyl- (außer Methyl) oder eine Aminofunktion tragen, werden von dem von Ohta beschriebenen Wildtypenzym nicht umgesetzt.

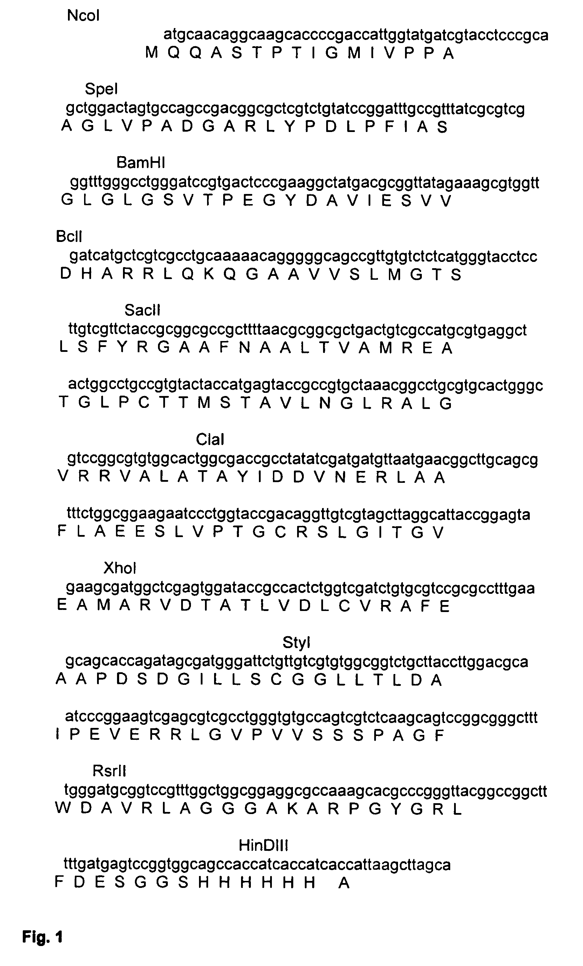

In Figur 1 sind die Wildtyp-Aminosäuresequenz der Arylmalonat-Decarboxylase aus Alcaligenes bronchisepticus (SEQ ID NO:1) und die hierfür kodierende Nucleotidsequenz (SEQ ID NO:2) dargestellt.

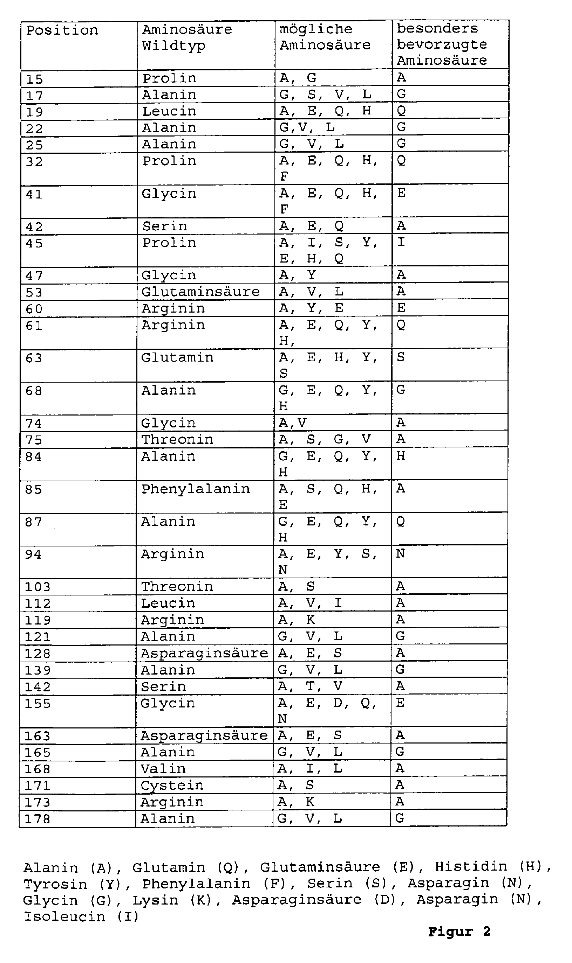

In der Figur 2 sind die bevorzugten Aminosäureaustausche dargestellt. Die jeweiligen Positionen beziehen sich auf die Aminosäuresequenz, wie in SEQ ID NO:1 dargestellt. In der zweiten Spalte ist diejenige Aminosäure wiedergegeben, die bei der Wildtypsequenz auftritt. In der dritten Spalte sind diejenigen Aminosäuren im Einbuchstabencode genannt, die anstelle der im Wildtyp vorkommenden Aminosäure bevorzugt verwendet werden. In der vierten Spalte (ganz rechts) sind diejenigen Aminosäuren aufgeführt, die in besonders bevorzugter Ausführungsform an der jeweiligen Position anstelle der Wildtyp-Aminosäure in dem erfindungsgemäß veränderten Enzym eingesetzt werden.

Die Aminosäureaustausche führen zu folgenden Vorteilen: bessere Umsetzung für bereits bekannte Substrate; Umsetzung neuer Substrate, d.h. von solchen Substraten, die durch das Wildtyp-Enzym nicht umgesetzt werden und Verbesserung der Thermostabilität.

Die erfindungsgemäßen Decarboxylasen weisen wenigstens einen Aminosäureaustausch auf. Es ist aber durchaus bevorzugt, daß mehrere, also wenigstens zwei oder drei, in besonderen Fällen auch noch mehr Aminosäuren gleichzeitig ausgetauscht sind.

Die vorliegende Erfindung wird durch die nachfolgenden Beispiele weiter erläutert.

### Beispiel 1: Klonierung der AMDase

Die Wildtyp-DNA-Sequenz der Arylmalonat-Decarboxylase aus *Alcaligenes bronchisepticus* wurde in einer für *E.coli* codonoptimierten Sequenz, gensynthetisch aufgebaut. Die Sequenz ist wiedergegeben im Sequenzprotokoll der SEQ ID NO:2.

atgcaacaggcaagcaccccgaccattggtatgatcgtacctcccgcagctggactagtgccagccgacggcgctcgtctgtatc cggatttgccgtttatcgcgtcgggtttgggcctgggatccgtgactcccgaaggctatgacgcggttatagaaagcgtggttgatca tgctcgtcgcctgcaaaaacagggggcagccgttgtgtctctcatgggtacctccttgtcgttctaccgcggcgccgcttttaacgcg gcgctgactgtcgccatgcgtgaggctactggcctgccgtgtactaccatgagtaccgccgtgctaaacggcctgcgtgcactgg gcgtccggcgtgtggcactggcgaccgcctatatcgatgatgttaatgaacggcttgcagcgtttctggcggaagaatccctggta ccgacaggttgtcgtagcttaggcattaccggagtagaagcgatggctcgagtggataccgccactctggtcgatctgtgcgtccg cgcctttgaagcagcaccagatagcgatgggattctgttgtcgtgtggcggtctgcttaccttggacgcaatcccggaagtcgagc gtcgcctgggtgtgccagtcgtctcaagcagtccggcgggcttttgggatgcggtccgtttggctggcggaggcgccaaagcacg cccgggttacggccggctttttgatgagtccggtggcagc

Der Aufbau des Genes erfolgte über Restriktions-/Ligations-Klonierung im Plasmid pUC19 zwischen den Schnittstellen EcoRI und HindIII. Dafür wurde die DNA-Sequenz am 3'Ende mit einem kurzen Linkerfragment mit der Sequenz 5'-ccggaattcccatgggccaa-3' (SEQ ID NO:3), der die EcoRI und die Ncol-Schnittstelle beinhaltet, und am 5' mit einem Linker der Sequenz 5'-caccatcaccatcaccattaagctt-3' (SEQ ID NO:4) versehen. Der Linker am 5'-Ende beinhaltet neben der Hindlll-Schnittstelle gleichzeitig die Codons für sechs Histidine, so dass die Decarboxylase über eine Einschrittreinigung mittels Metallaffinitätschromatographie erhalten werden kann.

Für die Klonierung der Gesamtsequenz wurden die Einzelstränge nach der Phosphoamidit-Methode synthetisiert und unter Standardbedingungen, nach Herstelleranleitung (New-England Biolabs), mit T4-Polynucleotidkinase phosphoryliert. Nach Hybridisierung der Einzelstränge zu entsprechenden DNA-Doppelsträngen wurden die Fragmente zum Gesamtgen zusammengesetzt. Nucleotidsequenz und Aminosäuresequenzen sind in Fig. 1 dargestellt.

Durch Sequenzierung des Klonierungsplasmids wurde die Sequenz überprüft. Zur Expression der Arylmalonat-Decarboxylase (AMDase) wurde die DNA-Sequenz in den pBAD-Vektor (Invitrogen) überführt (NcoI/HindIII) und in *E.coli* Zellen (Stamm: TOP10) eingebracht. Die Expression erfolgte durch Induktion der Bakterienzellen, bei einer optischen Dichte von - 0.6 (600nm), mit 0,02 % L-Arabinose für 8 bis 15 h bei 37 °C. Auch eine längere Induktion führt nicht zur Bildung von unlöslichem Protein in Form von *inclusion* bodies, sondert steigert die Ausbeute an löslichem Enzym weiter.

### Beispiel 2: Bereitstellung der AMDase

### 2.1 Fermentation der AMDase

Die Fermentation der Einzelmutanten, als auch des Wildtypproteins, erfolgte in LB-Medium mit Carbenicillin (200 µg/ml) als Antibiotikum (für den Laborfermenter konnte aber auch Ampicillin in gleicher Konzentration verwendet werden). Die Anzucht der Bakterienzellen (TOP10) erfolgt in 500 ml-Schüttelkolben (200 ml Kulturflüssigkeit) oder 5 I-Kolben (1 I Kulturflüssigkeit) bei 37°C bei ca. 300 rpm. Zur Expression der Proteine werden die Zellen mit 0,02 % (L)-Arabinose induziert und weiter bei 37°C geschüttelt. Die Proteinexpression kann sowohl bei 0,6 OD₆₀₀ gestartet werden, als auch direkt mit dem Animpfen der Kolben begonnen werden. Die Einzelkulturen wurden in der Regel für mindestens 4-6 h induziert.

Aus 200 ml Schüttelkultur lassen sich, je nach Mutation, Medium und Induktionsdauer, zwischen 5 und 150 mg/Liter Kulturmedium aufgereinigte AMDase isolieren.

Die AMDase-exprimierenden Zellen können aber auch im Laborfermenter angezogen werden.

Aus einer 4 Liter LB-Fermentation (pH-kontrolliert, mit Druckluft begast, ca 800 rpm Rührgeschwindigkeit, ca. 15 h Anzucht bei 37°C, Induktion bei animpfen mit 0,02 % L-Arabinose) lassen sich ca. 60 mg/ Liter aufgereinigtes Protein isolieren. Wenn die Zellen entsprechend in TB/Glycerin-Medium im Fermenter (pH-kontrolliert, mit Druckluft begast, ca 800 rpm Rührgeschwindigkeit, ca. 15 h Anzucht bei 37°C, Induktion bei animpfen mit 0,02 % L-Arabinose) angezogen werden, lassen sich leicht bis zu 240 mg Enzym/Liter Kulturmedium aufreinigen.

Im Vergleich gibt Ohta einen Ertrag von 1800 Units/Liter Kulturmedium (TB-Medium) an, was einer Ausbeute von knapp 5 mg reinem Enzym pro Liter entspricht (377 Units/mg Protein) [Ohta, Advances in Biochemical Engineering/Biotechnology vol. 63, pp1-30, 1999]

### 2.2 AMDase Aktivitätsassay auf Filterplattenbasis

Die mit Expressionsplasmid transformierten Bakterien werden auf LB-Agar, der, neben dem Antibiotikum Carbenicillin, 0,2 % Arabinose enthält, ausplattiert. Nach Anzucht, über Nacht im Brutschrank bei 37°C, werden die Bakterienkolonien auf sterile Papierrundfilter überstempelt und diese Filter im Anschluss daran an der Luft getrocknet. AMDase exprimierende Bakterienkolonien führen bei Inkubation der Filter mit 20 mM Substratlösung (pH6,0), der 0,5 mg/ml Bromthymolblau als pH-Indikator enthält, zu einer Verfärbung der Filterpartien von gelb (pH 6,0) nach blau (pH 8,0), so dass die Einzelkolonien entsprechend ihrer enzymatischen Aktivität unterschieden werden können. Dieses System kann vor allem verwendet werden, wenn Mutanten der AMDase bezüglich ihrer Substratspezifität untersucht werden. Die feine Unterscheidung von Aktivitätsunterschieden gelingt jedoch mit diesem Filterplattenassay nur ungenau.

### 2.3 Aufreinigung der AMDase

Zur Aufreinigung der AMDase und der AMDase-Mutanten wurden die induzierten Bakterien in 20 mM Tris/HCI pH 8,0 (5 ml Puffer/100 ml Kultur) über Ultraschall (15 min 70 %, auf Eis) aufgeschlossen. Die Zelldebris wurde durch Zentrifugation (30 min 10 000 g) entfernt (Überstand entspricht Nativ-Extrakt) und die resultierende lösliche Fraktion über NTA-Affinitätschromatographie (Qiagen) aufgereinigt. Dabei zeigte sich, dass die produzierte AMDase, bzw. die verschiedenen Mutanten vollständig als cytoplasmatisch-lösliches Protein exprimiert werden.

Die aktiven Fraktionen werden nach dem Auswaschen der nicht-aktiven Fraktionen (mit 50 mM Imidazol) mit 250 mM Imidazol vom Säulenmaterial eluiert, vereinigt und gegen Dialysepuffer (10 mM Kaliumphosphat pH 6.0, 0.1 mM EDTA, 5 mM β-Mercaptoethanol) umgesalzen. Die Lagerung der Mutanten kann als Proteinlösung gelöst bei 4°C erfolgen.

Eine Lagerung als Glycerinlösung (1:1 Mischungen der Proteinlösung (in Dialysepuffer) mit 87 % Glycerin) bei -20 oder -80 °C ist ebenfall gut möglich. Zur Umsetzung der getesten Substrate ist eine Abtrennung des zugesetzten Glycerins nicht nötig.

Es ist aber auch möglich, die Gesamtzellen, die AMDase exprimiert haben, mit Hilfe von Acetonwaschungen und anschliessendem Trocknen der Zellen, haltbar zu machen, und das Enzym zu einen späteren Zeitpunkt zu isolieren, oder die Gesamtzellen für eine Enzymumsetzung einzusetzten. Eine Haltbarmachung der Zellen kann auch eine Gefrieroder Sprühtrocknung mit umfassen.

### Beispiel 3: Charakterisierung der AMDase

### 3.1 Ermittlung des Umsatzes sowie des ee-Wertes

Die Ermittlung des Umsatzes sowie der erreichten Enantiomerenüberschüsse erfolgte nach bekannten Literaturangaben von [Miyamoto, K. & Ohta, H. (1992): Eur.J.Biochem. 210, 475-481].

### 3.2 Enzymkinetik der AMDase

Die enzymatische Aktivität des aufgereinigten Enzyms wurde in einem photometrischen Assay überprüft. Dazu wurde das entsprechende Substrat 20 mM in Wasser aufgelöst und mit KOH auf pH 6,0 eingestellt. Zur Substratlösung wurden zusätzlich 0,05 mg/ml

Bromthymolblau als Indikator hinzugegeben. Durch die Zugabe der AMDase kommt es zu einer Decarboxylierung des Substrates, die daraus resultierende pH-Änderung führt zu einem Farbumschlag des Indikators (gelb [pH 6,0] → blau [pH 8]), der bei 615 bis 620 nm im Photometer vermessen werden kann (**pH-Assay**). Dabei reicht es aus, 200 µl Substratlösung mit 20 µl einer Enzymlösung, die eine optische Dichte von 0,15 aufweist (ca. 5,5 µg, Bradford-Test mit BSA als Referenz), zu versetzen.

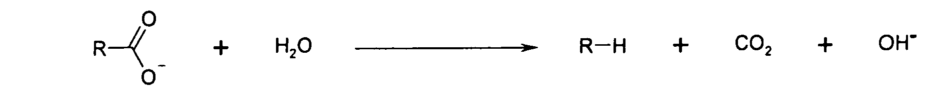

Neben der Phenylmalonsäure wurden noch eine Reihe weiterer Malonsäurederivate hergestellt und untersucht.

### 3.3 Umsetzung von Phenylmalonsäure unter Verwendung ganzer Zellen

Für die Substratumsetzung mit ganzen Zellen wurden Zellen, die das enzymkodierende Plasmid tragen, 6 h unter enzym-induzierenden Bedingungen angezogen (0,02 % Arabinose in LB-Medium). Zu den induzierten und sedimentierten Zellen wurde Substratlösung (20 mM Phenylmalonsäure pH 6.0, 0.05 mg/ml Bromthymolblau) zugefügt und die Extinktionsänderung am Photometer über die Zeit aufgezeichnet.

Bei der Umsetzung von Phenylmalonsäure mit ganzen, AMDase produzierenden Zellen wurde festgestellt, daß sowohl cytoplasmatische Expression wie auch periplasmatische Expression für eine Umsetzung des Substrates einsetzbar ist.

### 3.4 Umsetzung unterschiedlicher Substrate unter Verwendung von AMDase nach periplasmatischer Expression.

Für die Substratumsetzung mit ganzen Zellen wurden Zellen, die das enzymkodierende Plasmid einschließlich der N-terminalen *pelB* Exportsequenz tragen, 6 h unter enzym-induzierenden Bedingungen angezogen (0,02 % Arabinose in LB-Medium). Zu den induzierten und sedimentierten Zellen wurde Substratlösung (20 mM Phenylmalonsäure pH 6.0, 0.05 mg/ml Bromthymolblau) zugefügt und die Extinktionsänderung am Photometer über die Zeit aufgezeichnet (vgl. oben).

**PelB AMDase (Wildtyp) N-Terminus: MKTLLPTAAAGLLLLAAEPAMAMGQMQQASTPTIGMI.....** (SEQ ID NO:6)

Die periplasmatische Proteinexpression kann über eine Periplasmapräparation verifiziert werden.

### Beispiel 4: AMDase Mutanten mit verbesserter Aktivität gegenüber den bisher bekannten Substraten

Durch Mutagenese wurden zahlreiche Mutanten der AMDase erzeugt, die eine verbesserte Enzymaktivität gegenüber den bisher bekannten Substraten von Ohta et. al. aufweisen. Die veränderten Aminosäuresequenzen wurden durch Sequenzierung der Expressionsplasmide verifiziert. Dabei hat sich gezeigt, dass der Austausch bestimmter Aminosäuren, wie zum Beispiel 17, 19, 22, 25, 32, 41, 42, 45, 47, 53, 60, 61, 63, 68, 74, 84, 85, 87, 94, 103, 112, 119, 121, 139, 142, 155, 168, 173, und/oder 178 gegen Alanin bzw. Glycin oder weiterer Aminosäuren, wie zum Beispiel Glutamin oder Glutaminsäure und Histidin zu einer verbesserten Umsetzung, von α-Methyl-Phenylmalonsäurederivaten bzw. der Phenylmalonsäure zu α-Methyl-Phenylessigsäurederivaten bzw. Phenylessigsäure führt. Bei den Mutationen wurde ein Wildtyp Alanin durch Glycin oder Glutamin, Glutaminsäure oder Histidin bzw. eine andere Aminosäure durch Alanin oder weitere Aminosäuren ersetzt.

### Beispiel 5: AMDase Mutanten mit verbesserter Aktivität gegenüber α-Hydroxy-, α-Thiol- bzw. α-Amino-Phenylmalonsäurederivaten am Beispiel der α-Hydroxy-(4-Methylphenyl)-malonsäure.

Durch Mutagenese wurden zahlreiche Mutanten der AMDase erzeugt, die eine verbesserte Enzymaktivität gegenüber α-Hydroxy-Phenylmalonsäurederivaten aufweisen. Die veränderten Aminosäuresequenzen wurde durch Sequenzierung der Expressionsplasmide verifiziert. Dabei hat sich gezeigt, dass der Austausch bestimmter Aminosäuren, wie zum Beispiel von Phenylalanin 85 gegen Alanin (F85A), zu einer Umsetzung von α-Hydroxy-(4-Methylphenyl)-malonsäure (KS38) zu dem entsprechenden Mandelsäurederivat, in hoher Ausbeute (94 %) und mit hohem ee-Werte (98) führt. Dabei war es unerheblich, ob beispielsweise das aromatische Ringsystem in *para* Stellung eine Methyl- oder eine Isobutyryl-Gruppe trägt (Vergleich KS38/ KS30). Aber auch die Einführung verschiedener anderer Mutationen wie z.B. an Position 17, 19, 32, 41, 42, 45, 47, 53, 60, 61, 63, 68, 74, 84, 85, 87, 94, 103, 119, und/oder 142 (Einzelmutationen wie auch Doppel- und Dreifachmutationen oder Mehrfachmutationen) führt zu einer Umsetzung von α-Hydroxy-Phenylmalonsäurederivaten.

Die eingesetzten Substrate weisen folgende Strukturformeln auf:

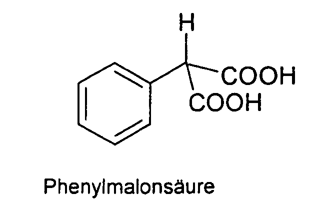

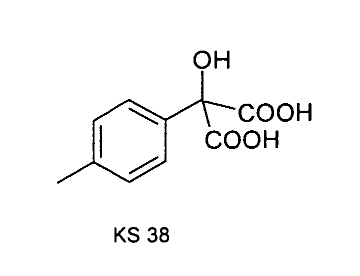

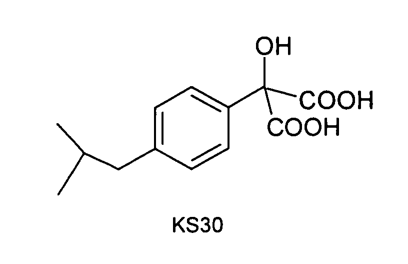

Die entsprechenden α-Methyl-Phenylmalonsäuren wurden nach bekannter Synthesevorschrift hergestellt und eingesetzt [Ghosh, S. et al. (1982): J.Org.Chem. 47, 4692-4702]. Die zu diesem Syntheseweg (der enzymatischen Decarboxylierung) nötigen Ausgangsverbindungen erhält man, indem man ein α-Keto-Malonsäureester über Zinn(IV)chlorid mit der entsprechend einzuführenden Arylgruppe umsetzt.

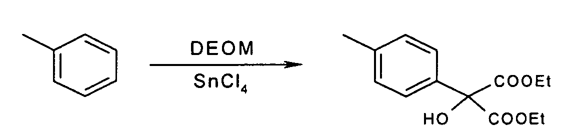

Nach Verseifung des Carbonsäureester (chemisch oder enzymatisch) kann das resultierende α-Hydroxymalonsäurederivat direkt als Enzymsubstrat verwendet werden [Miyamoto, K. & Ohta, H. (1991): Biocatalysis 5, 49-60].

Für die enzymatische Spaltung der Carbonsäureester wären zwei Wege denkbar. Zum einen eine vorgeschaltete Hydrolyse der Carbonsäureester mit Hilfe einer Schweineleber-Esterase oder einer unspezifischen Hydrolase [Faber, K., Biotransformations in Organic Chemistry, 4th Ed., Springer-Verlag (2000); Drauz, K., Waldmann, H., Enzym Catalysis in Organic Synthesis, VCH (1995); Lange et al., Chembiochem. **2**, 576-82 (2001], so dass die resultierende Dicarbonsäure dann zur Decarboxylierung eingesetzt werden kann. Es wäre aber auch eine Koexpression der Decarboxylase zusammen mit dem Ester-spaltenden Enzym im gleichen Wirtsorganismus möglich, dies würde dann bedeuten, dass nur ein Einschrittreaktionsverfahren durchgeführt werden muss.

Als Beispiele sind Ergebnisse der enzymatischen Decarboxylierung in Tabelle 1 zusammengefaßt.

**Tabelle 1**

| **Zusammenfassung einiger Ergebnisse am Beispiel der Phenylmalonsäure und von KS38.** | | | | |
|---|---|---|---|---|
| Position der Mutation | Aktivität Phenylmalonsäure (ΔE/min) | Relative Verbesserung Phenylmalonsäure | Aktivität KS38 (ΔE/min) | relative Verbesserung KS38 |
| Wildtyp-Enzym | 0,0957 | 1 | 0,00162 | 1 |
| A84G | 0,2051 | 2,14 | 0,00323 | 1,99 |
| F85A | 0,1925 | 2,01 | 0,00515 | 3,18 |
| A87G | 0,1477 | 1,54 | 0,00366 | 2,26 |
| R94A | 0,1592 | 1,66 | 0,00371 | 2,29 |
| T103A | 0,1661 | 1,73 | 0,00408 | 2,52 |
| I127A | 0,1850 | 1,93 | 0,00410 | 2,53 |
| F85A,R173A | 0,1361 | 1,42 | 0,00508 | 3,14 |
| F85A, E176A | 0,1091 | 1,14 | 0,00520 | 3,21 |
| F85A, A178G | 0,1134 | 1,18 | 0,00653 | 4,03 |

In der ersten Spalte der Tabelle 1 unter der Überschrift "Position der Mutation" ist die Lokalisierung der Mutation angegeben. Der erste Großbuchstabe gibt die Aminosäure in der Wildtypsequenz an der Position an, die durch die nachfolgende Zahl (z.B. 84) gekennzeichnet ist. Der zweite Großbuchstabe gibt die Aminosäure an, die bei der Mutante anstelle der Wildtyp-Aminosäure eingefügt wurde. So bedeutet beispielsweise "A84G", daß das Alanin an Position 84 der Wildtypsequenz ersetzt wurde durch ein Glycin in der Mutantensequenz. Mehrere derartige Angaben bedeuten, daß die Sequenz mehrere Mutationen aufweist, so beschreiben die letzten drei Zeilen der Tabelle I Doppelmutationen.

### Beispiel 6: AMDase Mutanten mit verbesserter Stabilität gegenüber chemischer bzw. thermischer Denaturierung.

Durch Mutagenese, wurden zahlreiche Mutanten der AMDase erzeugt, die eine erhöhte Stabilität gegenüber chemischer bzw. thermischer Denaturierung aufweisen. Die veränderten Aminosäuresequenzen wurden durch Sequenzierung der Expressionsplasmide verifiziert. Dabei hat sich gezeigt, dass der Austausch bestimmter Aminosäuren, wie zum Beispiel von P15 gegen Alanin (P15A), der Austausch von G74 gegen Alanin (G74A) und D128 gegen Alanin (D128A), zu einer deutlichen physikalischen Stabilisierung des Enzyms führen.

### Vergleich einiger durch Mutagenese stabilisierter Mutanten der AMDase

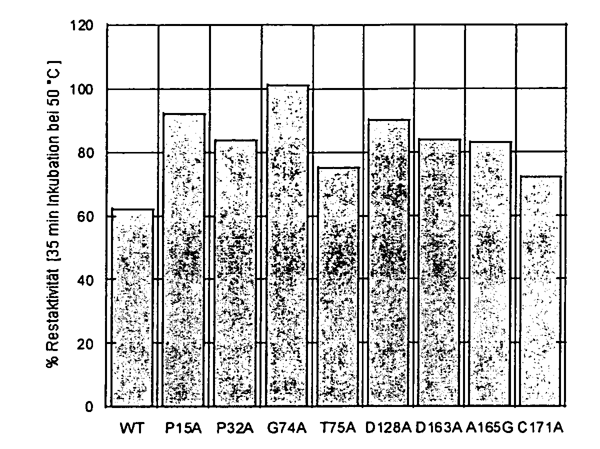

Tabelle 2: Restaktivität des Wildtypproteins sowie einiger neu generierter Mutanten, nach Inkubation bei 50°C für 35 min (10 mM Kaliumphosphat pH 6.0, 0.1 mM EDTA, 5mM β-Mercaptoethanol). Anschließend wurde die Aktivität im Vergleich zum unbehandelten Enzym anhand der Umsetzung der Phenylmalonsäure untersucht (20mM pH 6.0). Bei den hier gezeigten Mutanten handelt es sich um: P15A, P32A, G74A, T75A, D128A, D163A, A165G und C171A.

## Patentansprüche

1. Verfahren zur stereoselektiven Decarboxylierung von Malonsäurederivaten der Formel (I) 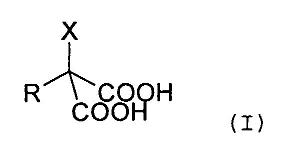 worin X die Bedeutung OH, SH, C₂-C₁₀ Alkyl, C₁-C₁₀ Alkyloxy, NH₂ hat und R die Bedeutung substituierte und nicht-substituierte Aryl-, Heteroaryl-, kondensierte Aryl- sowie kondensierte Heteroarylrest darstellt, wobei der Substituent ein Halogen, Alkyl-, Alkenyl-, Alkinylrest sein kann, zu einer Verbindung der Formel (II) 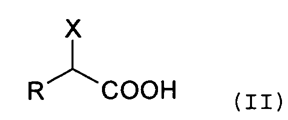 wobei X und R die oben angegebene Bedeutung haben mit einer Decarboxylase.

2. Decarboxylase enthaltend eine Aminosäuresequenz entsprechend der SEQ ID NO:1, die wenigstens eine Mutation gegenüber der SEQ ID NO:1 aufweist, wobei die Mutuation an wenigstens einer der folgenden Aminosäurepositionen vorliegt: 17, 19, 22, 25, 32, 41, 42, 45, 47, 53, 60, 61, 63, 68, 74, 84, 85, 87, 94, 103, 112, 119, 121, 139, 142, 155, 168, 173, und/oder 178, wobei die an der Aminosäureposition in SEQ ID NO:1 angegebene Aminosäure ersetzt ist durch eine beliebige andere Aminosäure, mit der Maßgabe, daß an der Position 188 der Cysteinrest nicht ersetzt sein darf, sowie Insertions- und/oder Deletionsmutanten, wobei nicht mehr als jeweils 10 Aminosäuren in die Sequenz gemäß SEQ ID NO:1 eingefügt und/oder von ihr deletiert sein dürfen.

3. Decarboxylase nach Anspruch 2, **dadurch gekennzeichnet, daß** die Aminosäuresequenz wenigstens eine Mutation gegenüber der SEQ ID NO:1 aufweist und die Mutation an folgenden Aminosäurepositionen vorliegt: 17, 19, 32, 41, 42, 45, 47, 53, 60, 61, 63, 68, 74, 84, 85, 87, 94, 103, 119, und /oder 142,

4. Decarboxylase nach einem der Ansprüche 2 oder 3, **dadurch gekennzeichnet, daß** die Mutation an wenigstens einer der folgenden Positionen vorliegt: 19, 32, 42, 45, 47, 60, 61, 63, 68, 84, 85, und/oder 103 und, daß die in SEQ ID NO:1 an dieser Position angegebene Aminosäure ersetzt ist.

5. Decarboxylase nach einem der Ansprüche 2 bis 4, **dadurch gekennzeichnet, daß** die Aminosäuresequenz wenigstens eine Mutation zur Verbesserung der Thermostabilität aufweist, wobei die in SEQ ID NO:1 angegebene Aminosäure an den Positionen 15, 32, 74, 75, 128, 163, 165 und/oder 171 ersetzt ist.

6. Decarboxylase nach einem der Ansprüche 2 bis 5 zur Umsetzung von Malonsäure-Derivaten der Formel III 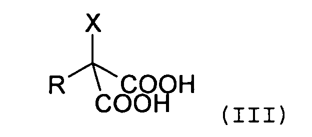 zu einer Verbindung der Formel (IV) 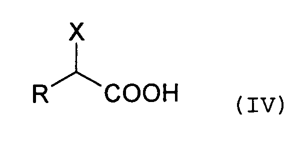 worin X die Bedeutung F, CH₃ oder H hat und R die Bedeutung substituierte und nicht-substituierte Aryl-, Heteroaryl-, kondensierte Aryl- sowie kondensierte Heteroarylrest darstellt, wobei der Substituent ein Halogen, Alkyl-, Alkenyl-, Alkinylrest sein kann, wobei wenigstens eine der in SEQ ID NO:1 angegebenen Aminosäuren an den Positionen 17, 19, 22, 25, 32, 41, 42, 45, 47, 53, 60, 61, 63, 68, 74, 84, 85, 87, 94, 103, 112, 119, 121, 139, 142, 155, 168, 173, und/oder 178 der Decarboxylase ersetzt ist durch eine in SEQ ID NO:1 an dieser Position nicht vorgegebene Aminosäure.

7. Decarboxylase nach einem der Ansprüche 2 bis 6, **dadurch gekennzeichnet, daß** der Cysteinrest an der Position 188 der SEQ ID NO:1 nicht mutiert ist und die bei der stereoselektiven Decarboxylierung entstehenden Verbindungen der Formel (II) die R-Konfiguration aufweisen.

8. Decarboxylase nach einem der Ansprüche 2 bis 7, **dadurch gekennzeichnet, daß** eine Aminosäure zwischen den Positionen 69 und 81 der SEQ ID NO:1 durch ein Cystein ersetzt ist und gleichzeitig der Cysteinrest an Position 188 ersetzt ist durch eine andere Aminosäure als Cystein und, daß die bei der stereoselektiven Decarboxylierung entstehende Verbindung der Formel (II) die S-Konfiguration aufweist.

9. Decarboxylase nach einem der Ansprüche 2-8, **dadurch gekennzeichnet, daß** sie am C-Terminus und/oder am N-Terminus weitere Aminosäuren aufweist, insbesondere eine Polyhistidinsequenz am C-Terminus oder N-Terminus.

10. Verfahren zur Herstellung einer Decarboxylase nach einem der Ansprüche 2-9, **dadurch gekennzeichnet, daß** in einem geeigneten Wirtsorganismus mit einem Vektor, der die Polynucleotidsequenz ID NO:2 mit den für die Mutationen kodierenden Basenaustauschen enthält, und/oder der das Gen mit regulatorischen Einheiten chromosomal integriert enthält, die Decarboxylase exprimiert sowie gegebenenfalls gereinigt wird.

11. Verfahren nach Anspruch 10, **dadurch gekennzeichnet, daß** der Wirtsorganismus Escherichia coli ist.

12. Transformierter Wirtsorganismus, **dadurch gekennzeichnet, daß** er eine Decarboxylase nach einem der Ansprüche 2 bis 9 exprimieren kann.


SEQUENZPROTOKOLL

<110> BioSpring GmbH

<120> Enzymatische asymmetrische Decarboxylierung von
      disubstituierten Malonsäuren

<130> decarbox

<140>
<141>

<160> 6

<170> PatentIn Ver. 2.1

<210> 1
<211> 250
<212> PRT
<213> Alcaligenes bronchisepticus

<400> 1
Met Gln Gln Ala Ser Thr Pro Thr Ile Gly Met Ile Val Pro Pro Ala
  1               5                  10                  15

Ala Gly Leu Val Pro Ala Asp Gly Ala Arg Leu Tyr Pro Asp Leu Pro
             20                  25                  30

Phe Ile Ala Ser Gly Leu Gly Leu Gly Ser Val Thr Pro Glu Gly Tyr
         35                  40                  45

Asp Ala Val Ile Glu Ser Val Val Asp His Ala Arg Arg Leu Gln Lys
     50                  55                  60

Gln Gly Ala Ala Val Val Ser Leu Met Gly Thr Ser Leu Ser Phe Tyr
 65                  70                  75                  80

Arg Gly Ala Ala Phe Asn Ala Ala Leu Thr Val Ala Met Arg Glu Ala
                 85                  90                  95

Thr Gly Leu Pro Cys Thr Thr Met Ser Thr Ala Val Leu Asn Gly Leu
            100                 105                 110

Arg Ala Leu Gly Val Arg Arg Val Ala Leu Ala Thr Ala Tyr Ile Asp
        115                 120                 125

Asp Val Asn Glu Arg Leu Ala Ala Phe Leu Ala Glu Glu Ser Leu Val
    130                 135                 140

Pro Thr Gly Cys Arg Ser Leu Gly Ile Thr Gly Val Glu Ala Met Ala
145                 150                 155                 160

Arg Val Asp Thr Ala Thr Leu Val Asp Leu Cys Val Arg Ala Phe Glu
                165                 170                 175

Ala Ala Pro Asp Ser Asp Gly Ile Leu Leu Ser Cys Gly Gly Leu Leu
            180                 185                 190

Thr Leu Asp Ala Ile Pro Glu Val Glu Arg Arg Leu Gly Val Pro Val
        195                 200                 205

Val Ser Ser Ser Pro Ala Gly Phe Trp Asp Ala Val Arg Leu Ala Gly
    210                 215                 220

Gly Gly Ala Lys Ala Arg Pro Gly Tyr Gly Arg Leu Phe Asp Glu Ser
225                 230                 235                 240

Gly Gly Ser His His His His His His Ala
                245                 250

<210> 2
<211> 769
<212> DNA
<213> Künstliche Sequenz

<220>
<223> Beschreibung der künstlichen Sequenz: Konstrukt

<400> 2
ccatgggcca aatgcaacag gcaagcaccc cgaccattgg tatgatcgta cctcccgcag 60
ctggactagt gccagccgac ggcgctcgtc tgtatccgga tttgccgttt atcgcgtcgg 120
gtttgggcct gggatccgtg actcccgaag gctatgacgc ggttatagaa agcgtggttg 180
atcatgctcg tcgcctgcaa aaacaggggg cagccgttgt gtctctcatg ggtacctcct 240
tgtcgttcta ccgcggcgcc gcttttaacg cggcgctgac tgtcgccatg cgtgaggcta 300
ctggcctgcc gtgtactacc atgagtaccg ccgtgctaaa cggcctgcgt gcactgggcg 360
tccggcgtgt ggcactggcg accgcctata tcgatgatgt taatgaacgg cttgcagcgt 420
ttctggcgga agaatccctg gtaccgacag gttgtcgtag cttaggcatt accggagtag 480
aagcgatggc tcgagtggat accgccactc tggtcgatct gtgcgtccgc gcctttgaag 540
cagcaccaga tagcgatggg attctgttgt cgtgtggcgg tctgcttacc ttggacgcaa 600
tcccggaagt cgagcgtcgc ctgggtgtgc cagtcgtctc aagcagtccg gcgggctttt 660
gggatgcggt ccgtttggct ggcggaggcg ccaaagcacg cccgggttac ggccggcttt 720
ttgatgagtc cggtggcagc caccatcacc atcaccatta agcttagca 769

<210> 3

<211> 20
<212> DNA
<213> Künstliche Sequenz

<220>
<223> Beschreibung der künstlichen Sequenz: Primer

<400> 3
ccggaattcc catgggccaa                                                    20


<210> 4
<211> 25
<212> DNA
<213> Künstliche Sequenz

<220>
<223> Beschreibung der künstlichen Sequenz: Primer

<400> 4
caccatcacc atcaccatta agctt                                              25


<210> 5
<211> 729
<212> DNA
<213> Alcaligenes bronchisepticus

<400> 5
atgcaacagg caagcacccc gaccattggt atgatcgtac ctcccgcagc tggactagtg 60
ccagccgacg gcgctcgtct gtatccggat ttgccgttta tcgcgtcggg tttgggcctg 120
ggatccgtga ctcccgaagg ctatgacgcg gttatagaaa gcgtggttga tcatgctcgt 180
cgcctgcaaa aacagggggc agccgttgtg tctctcatgg gtacctcctt gtcgttctac 240
cgcggcgccg cttttaacgc ggcgctgact gtcgccatgc gtgaggctac tggcctgccg 300
tgtactacca tgagtaccgc cgtgctaaac ggcctgcgtg cactgggcgt ccggcgtgtg 360
gcactggcga ccgcctatat cgatgatgtt aatgaacggc ttgcagcgtt tctggcggaa 420
gaatccctgg taccgacagg ttgtcgtagc ttaggcatta ccggagtaga agcgatggct 480
cgagtggata ccgccactct ggtcgatctg tgcgtccgcg cctttgaagc agcaccagat 540
agcgatggga ttctgttgtc gtgtggcggt ctgcttacct tggacgcaat cccggaagtc 600
gagcgtcgcc tgggtgtgcc agtcgtctca agcagtccgg cgggcttttg ggatgcggtc 660
cgtttggctg gcggaggcgc caaagcacgc ccgggttacg gccggctttt tgatgagtcc 720
ggtggcagc                                                                729


<210> 6
<211> 37
<212> PRT
<213> Künstliche Sequenz

<220>
<223> Beschreibung der künstlichen Sequenz:
      Exportsequenz

<400> 6
Met Lys Thr Leu Leu Pro Thr Ala Ala Ala Gly Leu Leu Leu Leu Ala
  1               5                  10                  15

Ala Glu Pro Ala Met Ala Met Gly Gln Met Gln Gln Ala Ser Thr Pro
             20                  25                  30

Thr Ile Gly Met Ile
         35